# EUROPÄISCHE PATENTSCHRIFT

(11) **EP 0 891 436 B1**
(45) Veröffentlichungstag und Bekanntmachung des Hinweises auf die Patenterteilung: **28.11.2001**
(21) Anmeldenummer: 97904982.2
(22) Anmeldetag: 12.03.1997
(51) Int. Cl.: D01H 13/22, D01H 13/26, B65H 63/06

(54) **VERFAHREN UND VORRICHTUNG ZUR QUALITÄTSÜBERWACHUNG VON GARNEN**
PROCESS AND DEVICE FOR MONITORING THE QUALITY OF YARNS
PROCEDE ET DISPOSITIF POUR SURVEILLER LA QUALITE DE FILS

(30) Priorität: 27.03.1996 CH 79296
(43) Veröffentlichungstag der Anmeldung: 20.01.1999
(73) Patentinhaber: ZELLWEGER LUWA AG, 8610 Uster (CH)
(72) Erfinder: ARB, Werner, CH-8610 Uster (CH); FÄRBER, Christoph, D-41352 Korschenbroich (DE)
(74) Vertreter: Ellenberger, Maurice
(86) Internationale Anmeldenummer: CH9700103
(87) Internationale Veröffentlichungsnummer: WO9736032

(56) Entgegenhaltungen:
- EP-A- 0 643 294
- EP-A- 0 652 432
- EP-A- 0 685 580
- CH-A- 674 379
- PATENT ABSTRACTS OF JAPAN vol. 00, no. 00 & JP 06 322621 A (MURATA MACH LTD), 22.November 1994,
- PATENT ABSTRACTS OF JAPAN vol. 12, no. 333 (C-526), 8.September 1988 & JP 63 092738 A (MURATA MACH LTD), 23.April 1988,

## Beschreibung

Die Erfindung betrifft ein Verfahren und eine Vorrichtung zur Qualitätsüberwachung von Garnen, wobei ein vom Garn abgeleitetes Signal in einem Klassierfeld klassiert wird.

Aus der CH-477573 ist beispielsweise eine Vorrichtung für Garnreiniger bekannt, mit der eine Qualitätsüberwachung von Garnen durchgeführt werden kann, indem Fehler, die bestimmte Grenzwerte überschreiten, herausgeschnitten werden. Ein Fehler wird dabei durch eine Über- oder Unterschreitung des mittleren Durchmessers des Garns über eine endliche Länge gekennzeichnet. Bestimmte Fehler werden durch ein Messer herausgeschnitten und das Garn anschliessend wieder verspleisst. Um ein Schneidmesser anzusteuern wird eine sogenannte Reinigungsgrenze festgelegt, an der alle Fehler gemessen werden. Diejenigen Fehler, die die Reinigungsgrenze überschreiten, werden durch das Schneidmesser herausgeschnitten. Die Reinigungsgrenze setzt sich aus Punkten zusammen, von denen jeder durch eine bestimmte Abweichung vom mittleren Durchmesser und eine bestimmte Länge eines Garnabschnittes gekennzeichnet ist. Bekannte Reinigungsgrenzen haben einen kontinuierlichen oder einen diskontinuierlichen Verlauf (mit Unstetigkeiten). Üblicherweise bewirken sie aber, dass grosse Abweichungen vom Durchmesser aber nur kurzer Länge nicht, geringe Abweichungen grosser Länge dagegen herausgeschnitten werden. Bekannt ist es, solche Reinigungsgrenzen über ein Klassierfeld zu legen, wobei die Klassengrenzen und die Reinigungsgrenze nicht immer übereinstimmen. Eine solche Reinigungsgrenze teilt aber den Raum oder eine Fläche immer in zwei Bereiche auf. Einen Bereich ausserhalb der Reinigungsgrenze für untolerierbare Fehler und einen Bereich innerhalb der Reinigungsgrenze für tolerierbare Fehler.

Ein Nachteil dieser bekannten Vorrichtungen ist beispielsweise darin zu sehen, dass neben den Unregelmässigkeiten des Garns an sich, auch Fremdstoffe, die im Garn eingesponnen sind und eine Durchmesseränderung bewirken, in gleicher Weise ausgereinigt werden wie gewöhnliche Dick- oder Dünnstellen im Garn. Das bedeutet, dass es nicht möglich ist, unter den verschiedenen vorkommenden Fremdstoffen wie Fremdkörper, Schalenteile, andersfarbige Fasern, Haare, Knoten usw. zu unterscheiden. Damit ist es auch nicht möglich selektiv einzelne Gattungen von Fremdstoffen herauszuschneiden. Bekannte Vorrichtungen sind mit Sensoren ausgestattet, die es erlauben, den Durchmesser oder die Masse eines Garnabschnittes zu erfassen und daraus die Gleichmässigkeit der Masse oder des Durchmessers über die Länge des Garns gesehen zu überwachen, oder auch allgemein Fremdstoffe im Garn zu erkennen. Im Sensor wird üblicherweise ein elektrisches Signal erzeugt, das einem Garnabschnitt zugeordnet ist. Daraus ist es aber schwierig und unsicher abzuleiten, ob im Garnabschnitt nun ein bestimmter Fremdkörper enthalten ist. Es ist möglich die Anwesenheit eines Fremdkörpers zu erkennen aber unmöglich die Art des Fremdkörpers zu bestimmen.

Aus der EP-A-0 685 580, die wie die CH-477 573 den am nächsten kommenden Stand der Technik darstellt, ist ein Verfahren und eine Vorrichtung bekannt, bei denen es darum geht, Signale, die den Durchmesser oder die Masse von Abschnitten eines Garns angeben, zu klassieren und zu zählen. Aus den Klassierungen und Zählwerten werden Hinweise gewonnen, die zur Überwachung oder Beeinflussung des Herstellungsprozesses des Garns dienen. Aus den Klassierungen und Zählwerten kann ein Bezug zur Ursache für einen Fehler im Garn hergestellt werden und ein Fehler im Herstellungsprozess behoben werden.

Ein Nachteil dieser weiteren Vorrichtung oder des Verfahrens ist beispielsweise darin zu sehen, dass damit nur Angaben über den Herstellungsprozess, nicht aber über die verarbeiteten Materialien gewonnen werden. So können Fehler im Herstellungsprozess zwar vermieden werden, es bleiben aber jene Fehler unbeeinflusst, die dem Rohmaterial zuzuschreiben sind. Ebenso wird nicht unterschieden, ob die im Garn erfassten Fehler durch Fremdstoffe verursacht sind oder andere materialbezogene Ursachen haben.

Es gibt somit keine Möglichkeit zwischen Garn und Fremdstoff und zwischen verschiedenartigen Fremdstoffen im Garn zu unterscheiden. Schalenteile im Garn werden üblicherweise durch die Weiterbearbeitung, z.B. die chemische Nachbehandlung zur Bleichung, aus dem Garn entfernt, denn Schalenteile sind später im gewobenen Tuch sehr gut erkennbar. Oftmals springen sie geradezu ins Auge oder machen sich dadurch bemerkbar, dass sie bei der Färbung Farbe nicht oder nur ungenügend annehmen.

Die Erfindung wie sie in den Patentansprüchen gekennzeichnet ist, löst demnach die Aufgabe, ein Verfahren und eine Vorrichtung zu schaffen, mit der bestimmte Fremdstoffe in einem Garnabschnitt einwandfrei vom Garn und anderen Fremdstoffen unterschieden werden können.

Diese Aufgabe wird durch die Verfahrensmerkmale des Anspruchs 1 und durch die Vorrichtungsmerkmale des Anspruchs 7.

Die durch die Erfindung erreichten Vorteile sind darin zu sehen, dass es damit möglich ist, eine Zuordnung zwischen dem Garn und einzelnen Arten von Fremdstoffen einerseits und dem vorliegenden elektrischen Signal andererseits vorzunehmen. Beispielsweise kann ausgehend von der Erkenntnis, dass Schalenteile besonders kurze Fehler ergeben, die Vorgabe gemacht werden, dass Fehler, die in einem oder mehreren Feldern klassiert sind, die den kürzesten Abschnitten zugeordnet sind, eben Schalenteile sein müssen. Ebenso kann vorgegeben werden, dass beispielsweise Fehler, die Feldern zugeordnet werden, die mittlere Längen und mittlere Durchmesseränderungen abgrenzen, Blatt- oder Stengelreste der Baumwollpflanze sein müssen usw.

Im folgenden wird die Erfindung anhand eines Beispiels und mit Bezug auf die beiliegenden Figuren näher erläutert. Es zeigen
- Figur 1: eine schematische Darstellung der erfindungsgemässen Vorrichtung,
- Figur 2: eine Darstellung eines Signales aus der Vorrichtung gemäss Fig. 1,
- Figuren 3 und 4: je eine Darstellung einer Klassiermatrix,
- Figur 5: ein Flussdiagram zum erfindungsgemässen Verfahren und
- Figur 6: eine Ansicht der Vorrichtung.

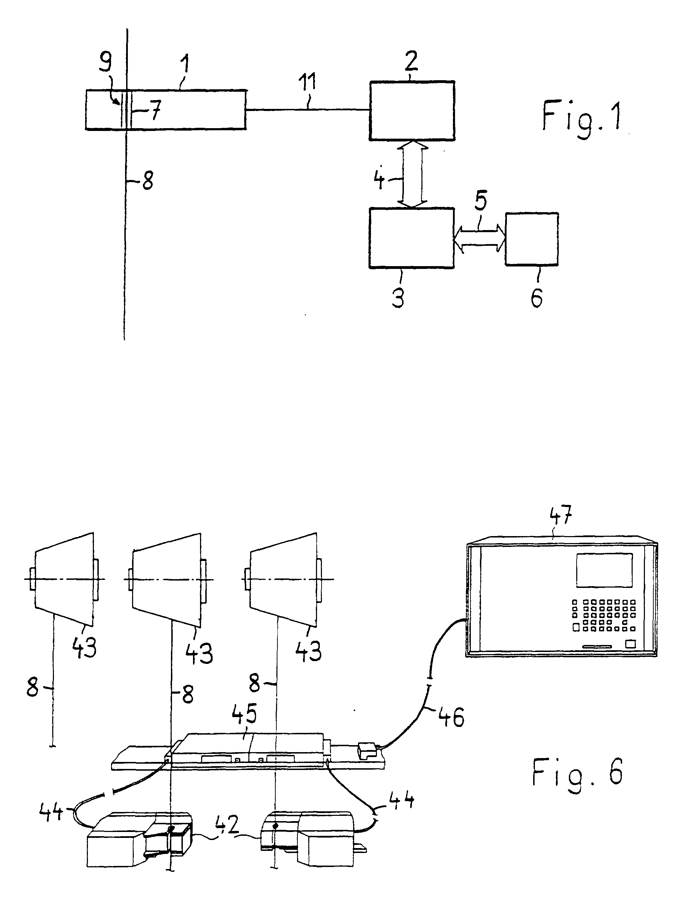

Die erfindungsgemässe Vorrichtung gemäss Fig. 1 besteht im wesentlichen aus einem Messkopf 1 an sich bekannter Bauart, einer Auswerteeinheit 2 und einem Steuergerät 3, das über einen Bus 4 mit der Auswerteeinheit 2 verbunden ist. Das Steuergerät 3 ist über einen weiteren Bus 5 mit einer Anzeige- und Bedienungseinheit 6 verbunden, die Teil einer Spinn- oder Spulmaschine sein kann. Der Messkopf 1 weist einen Messspalt 7 auf, in dem durch an sich bekannte und deshalb hier nicht näher dargestellte Mittel ein Garn 8 in seiner Längsrichtung hindurchbewegt und abschnittsweise detektiert wird. Vorzugsweise hat der Messkopf 1 ein Messsystem 9, das an den Messspalt 7 angrenzt und optisch, insbesondere mit Auflicht arbeitet. Das bedeutet, dass in bekannter Weise das am Garn reflektierte Licht in einem Sensor empfangen und zu einem elektrischen Signal gewandelt wird. Solche Messsysteme sind an sich bekannt und beispielsweise in der Patentanmeldung WO 93/13407 genauer beschrieben

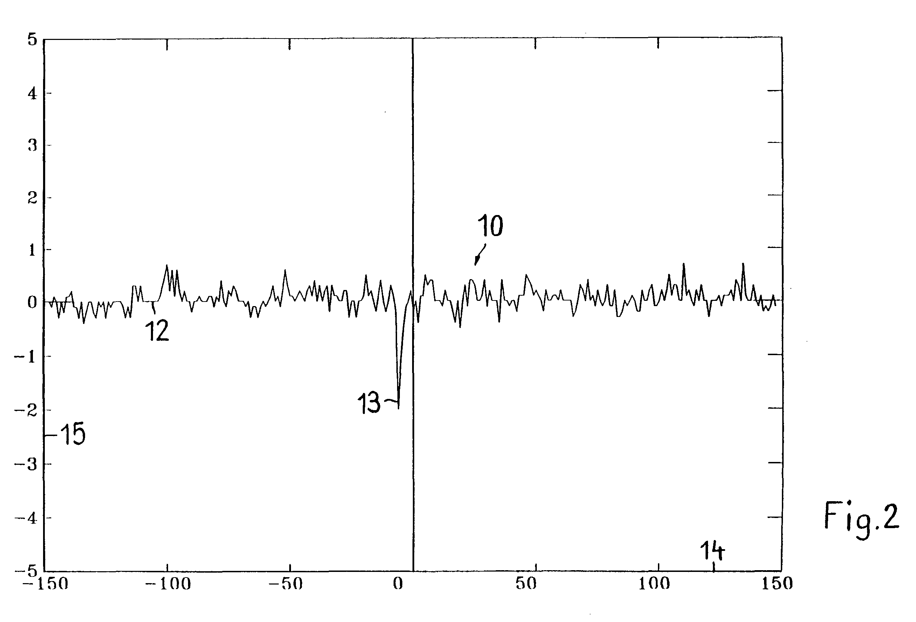

Fig. 2 zeigt ein elektrisches Signal 10 wie es vom Messkopf 1 beispielsweise über eine Leitung 11 (Fig. 1) abgegeben wird, die das Steuergerät 3 und den Messkopf 1 miteinander verbindet. Das Signal 10 ist über einer horizontalen Achse 14 aufgetragen, auf der Längeneinheiten angegeben sind, die sich auf das Garn 8 beziehen. Auf einer vertikalen Achse 15 sind beispielsweise Werte einer elektrischen Spannung aufgetragen. Dieses Signal 10 besteht im wesentlichen aus kleinen unbedeutenden Ausschlägen, die durch kleine Unregelmässigkeiten der Oberfläche des Garns 8 hervorgerufen sind. Man kann dies auch mit dem Begriff Rauschen zusammenfassen. Das Signal 10 ist um eine Nullinie 12 herum zentriert. Mit 13 ist eine herausragende Signalabweichung bezeichnet, die auf ein besonderes Ereignis schliessen lässt. Hier ist dieses Ereignis ein Schalenteil, das beispielsweise an einer Faser anhaftete und durch den Spinnvorgang in das Garn eingesponnen ist. Schalenteile sind feste Rückstände der Baumwollpflanze (z.B. Teile von Samen oder Samenkapseln), welche durch die mechanische Beanspruchung während des Verarbeitungsprozesses (z.B. beim Spinnen) in stark zerkleinerter Form im Garn vorliegen. Deren Grösse beträgt im Durchschnitt ca. 0.5 mm und deren Form ist kompakt, beispielsweise punktförmig. Die Signalabweichung ist hier beispielsweise durch eine verminderte Reflexion des genannten Auflichtes entstanden, die beispielsweise von der dunklen Farbe solcher Schalenteile herrührt.

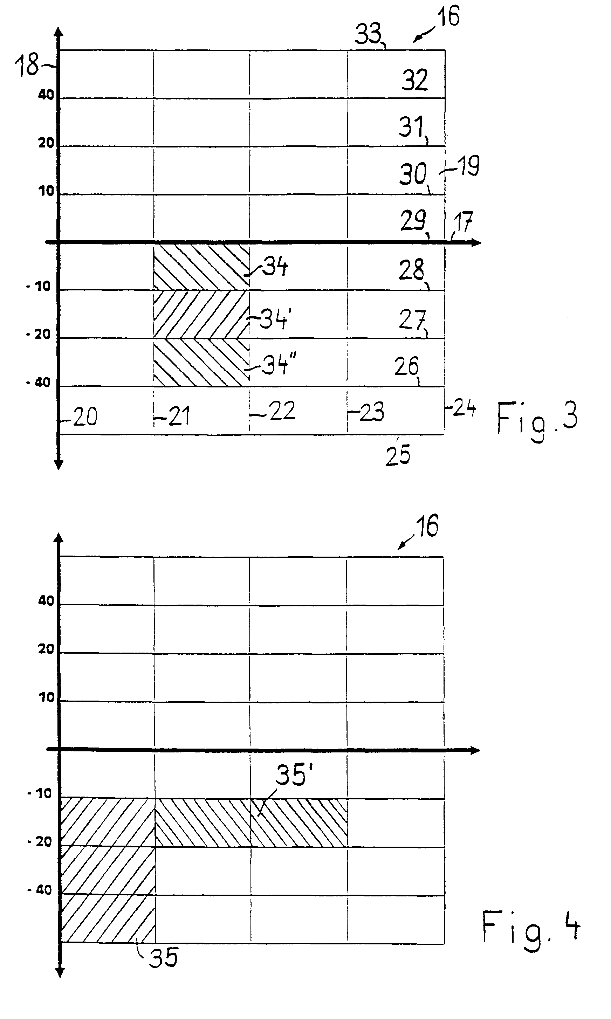

Fig. 3 zeigt ein Klassierfeld oder eine Klassiermatrix 16, die eine Achse 17 aufweist, längs der die Länge von Garnabschnitten in Millimetem aufgetragen ist, und die eine Achse 18 aufweist, längs der Werte für die Auslenkung oder Amplitude des elektrischen Signales 10 aufgetragen sind. Hier sind dies Werte, die das Mass der Reflexion in Prozenten angeben. Die gezeigte Klassiermatrix 16 weist 32 Felder 19 auf, die durch Linien 20 bis 24 und 25 bis 33 abgegrenzt sind. Diese Linien 20 bis 33 haben auch die Bedeutung von Schwellwerten, mit denen das Signal 10 verglichen wird. Ein einzelnes Feld 19 wie hier insbesondere Feld 34 wird immer durch vier Linien, hier die Linien 21, 22, 28 und 29 oder vier Schwellwerte abgegrenzt. Der Abstand der Linien 20 bis 24 voneinander kann beispielsweise 1 mm Länge auf dem Garn 8 entsprechen. Ein Feld oder eine Gruppe von Feldern, hier die Felder 34, 34' und 34" definiert Klassen für Signalabweichungen, die beispielsweise auf das Vorhandensein von Blatt- oder Stengelresten der Baumwolle im Garn hindeuten.

Fig. 4 zeigt nochmals das Klassierfeld 16 in dem ein Feld 35 eingetragen ist, das solche Längen und Auslenkungen des Signales 10 betrifft, die für Schalenteile charakteristisch sind. Signale die einem weiteren Feld 35' zugeordnet werden können, geben an, dass es sich bei der vorliegenden Verunreinigung um Jutereste einer Ballenverpackung handelt. Diese Jutereste sind üblicherweise zu Fasern aufgelöst.

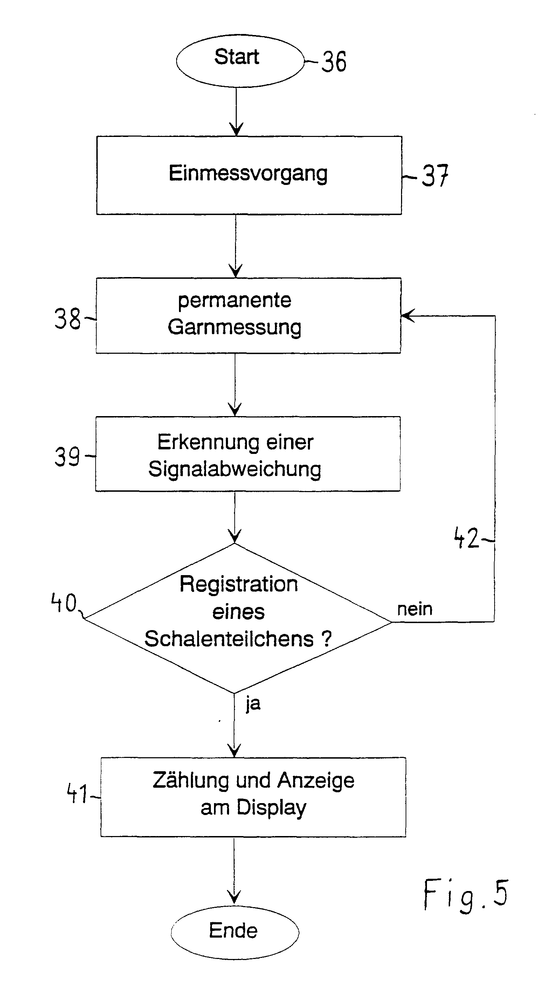

Fig. 5 zeigt ein Flussdiagramm für das erfindungsgemässe Verfahren. Damit soll nachfolgend die Arbeitsweise der Vorrichtung gemäss Fig. 1 erläutert werden. Das Verfahren wird beispielsweise durch Drücken einer Starttaste in der Bedienungseinheit 6 ausgelöst was durch den Schritt 36 bezeichnet ist. Das führt dazu, dass aus dem Steuergerät 3, welches beispielsweise aus einem Rechner (PC) besteht, das Programm in einen Prozessor in der Auswerteeinheit 2 geladen wird. Dann beginnt ein Einmessvorgang 37.

Der Einmessvorgang 37 wird durchgeführt, während ein Stück Garn von einigen Metern Länge durch den Messspalt 7 hindurch bewegt wird. Dies dient dazu erste Messwerte vom Garn 8, beispielsweise für die Reflexion zu gewinnen, daraus einen mittleren Wert zu berechnen und die Nullinie 12 (Fig. 2) darauf einzustellen. Dann beginnt die eigentliche Messung, die permanente Garnmessung 38.

Bei der permanenten Garnmessung 38 wird das Garn 8 durch den Messspalt 7 hindurchgezogen und es entsteht ein Signal 10 (Fig. 2). Das Signal 10 entsteht durch periodische Abtastung der kontinuierlich anfallenden Werte für die Reflexion des Lichtes im Messkopf 1. Jedesmal wenn sich das Garn 8 im Messspalt 7 um beispielsweise 2mm weiterbewegt hat, wird wieder ein Messwert erfasst und dieser wird mit allen Schwellwerten des Klassierfeldes 16 also mit allen Werten, die durch die Linien 20 bis 33 dargestellt sind, verglichen. Dies ist die Erkennung einer Signalabweichung 39 und geschieht im Prozessor der Auswerteeinheit 2. Das Programm im Prozessor gibt aber noch weitere Vorgaben. Werden insbesondere Schwellwerte entsprechend den Linien 28 oder 30 überschritten, so werden die entsprechenden Signalabschnitte in einen Speicher im Prozessor eingelesen und verbleiben dort so lange, bis der neueste Wert diese Linien 28 oder 30 in der anderen Richtung wieder überschreitet. Die Zeit des Aufenthaltes eines solchen Wertes im Speicher entspricht einer Länge wie sie auf der Achse 17 in Fig. 3 aufgetragen ist und wird dann an Schwellwerten gemessen, die durch Linien 21 bis 24 dargestellt sind. Beispielsweise ergibt ein Signalabschnitt, der den Schwellwert gemäss Linie 31, nicht aber Linie 32 überschreitet und solange im Speicher verbleibt, dass Schwellwerte gemäss den Linien 21 und 22, nicht aber 23 überschritten sind, einen Garnfehler der in Feld 34 zu klassieren ist. Wird ein solcher Fehler entdeckt, so wird er registriert, indem beispielsweise ein Zähler aktiviert wird, der diesem Feld 34 zugeordnete Fehler zählt oder ein an den Messkopf 1 anschliessend angeordnetes Messer wird aktiviert. Dies sind die Vorgänge, die einem Schritt 40, der die Registrierung vollendet und einem Schritt 41 der eine Zählung betrifft zugeordnet sind. Die Rückführung 42 deutet weiter an, dass diese Vorgänge ständig und für jede Abtastung oder jeden Messtakt wiederholt werden.

Aufgrund der klassierten Signalabweichungen lassen sich weitere Fremdstoffe feststellen. So kann man davon ausgehen, dass Fremdstoffe die zum Garn oder zum Licht der Lichtquelle mit der sie beleuchtet werden wenig Kontrast aufweisen Klassen oder Felder belegen, die geringe Signalabweichungen betreffen. In Fig. 3 beispielsweise entsprechend den Feldern die zwischen der Achse 17 und der Linie 28 liegen. Als Beispiel kann ein hellfarbiges Polypropylen genannt werden, das aus einer Verpackung stammt. Signale von Fremdstoffen die entsprechend viel Kontrast erzeugen, sind in Feldern zwischen den Linien 25 und 27 zu finden und können beispielsweise Kleidungsresten mit kräftigen Farben zugeordnet werden.

Fig. 6 zeigt eine Ansicht einer erfindungsgemässen Vorrichtung bei der Messköpfe 42 für die Erkennung von Fremdstoffen direkt auf einer Spinnmaschine oder auf einer Spulmaschine angeordnet sind. Die Messköpfe 42 werden vom Garn 8 durchlaufen kurz bevor dieses auf einer Spule 43 aufgewickelt wird. Über Leitungen 44 sind die Messköpfe 42 mit einer Auswerteeinheit 45 verbunden, die wiederum über einen Bus 46 mit einer zentralen Steuer-, Bedien- und Anzeigeeinheit 47 für mehrere Messköpfe verbunden ist. Auf diese Weise und mit dieser Vorrichtung ist es möglich, die Art oder Gattung der in einem Garnabschnitt auftretenden Fremdstoffe schon auf der Spinn- oder Spulmaschine zu erkennen und aufgrund des Signales aus dem Messkopf voneinander zu unterscheiden.

## Patentansprüche

1. Verfahren zur Qualitätsüberwachung von Garnen, wobei ein vom Garn abgeleitetes Signal in einem Klassierfeld klassiert wird, **dadurch gekennzeichnet, dass** ein vom Garn (8) abgeleitetes Reflexions-Signal (10) in dem Klassierfeld (16) klassiert wird und dass, ausgehend von dieser Klassierung, Gattungen von Fremdstoffen im Garn identifiziert werden.

2. Verfahren nach Anspruch 1, **dadurch gekennzeichnet, dass** für Werte des elektrischen Signals ein Klassierfeld (16) vorgegeben wird, das je mehrere Schwellwerte (25 bis 33 und 20 bis 25) für die Amplitude und die Länge/Dauer eines Signalabschnittes des elektrischen Signales aufweist, dass das Signal mit allen Schwellwerten verglichen wird, dass Klassen (34) durch Kombinationen von vier Schwellwerten gebildet werden, dass für jeden Garnabschnitt festgestellt wird zu welcher Klasse er gehört und dass ausgehend von der Klassenzugehörigkeit, Garnabschnitte der gleichen Klasse ausgesondert werden.

3. Verfahren nach Anspruch 2, **dadurch gekennzeichnet, dass** die Aussonderung der Garnabschnitte durch deren Zählung erfolgt.

4. Verfahren nach Anspruch 2, **dadurch gekennzeichnet, dass** die Aussonderung der Garnabschnitte durch Herausschneiden erfolgt.

5. Verfahren nach Anspruch 1, **dadurch gekennzeichnet, dass** bestimmten Fremdstoffen bestimmte Klassen zugeordnet werden.

6. Verfahren nach Anspruch 1, **dadurch gekennzeichnet, dass** das Garn (8) optisch durch Auflicht abgetastet wird.

7. Vorrichtung zur Durchführung des Verfahrens nach Anspruch 1, mit einem Sensor (1) zur Abtastung der Oberfläche des Garns (8), einer Einrichtung zur Ausgabe eines elektrischen Signals (10) und einer Auswerteeinheit (2), in der je mehrere Schwellwerte für die Länge eines Garnabschnittes gespeichert sind und in der ein Vergleich zwischen einem Wert des elektrischen Signals und allen Schwellwerten laufend erfolgt, **gekennzeichnet durch**, einen optisch arbeitenden Sensor (1) in dem ein Signal **durch** Reflexion von Licht am Garn gewonnen wird, **durch** Schwellwerte für Längen und Durchmesser, die ein Mass für die Reflexion am Garn bilden, wobei die Auswerteeinheit zur Bestimmung von Gattungen von Fremdstoffen im Garn ausgehend von deren Klassierung ausgebildet und angeordnet ist.

8. Vorrichtung nach Anspruch 7, **dadurch gekennzeichnet, dass** der Sensor direkt an einer Spinnstelle angeordnet ist.

9. Vorrichtung nach Anspruch 7, **dadurch gekennzeichnet, dass** der Sensor direkt an einer Spulstelle angeordnet ist.

## Claims

1. Process for monitoring the quality of yarns, wherein a signal derived from the yarn is classified in a classifying field, **characterised in that** a reflection signal (10) derived from the yarn (8) is classified in the classifying field (16) and that types of foreign substances in the yarn are identified, starting from this classification.

2. Process according to claim 1, **characterised in that** a classifying field (16) for values of the electrical signal is predetermined, which classifying field (16) has, in each case, a number of threshold values (25 to 33 and 20 to 25) for the amplitude and the length/duration of a portion of the electrical signal, that the signal is compared with all the threshold values, that classes (34) are formed by combinations of four threshold values, that it is ascertained, for each portion of yarn, to which class it belongs, and that portions of yarn of the same class are separated out, starting from the class affiliation.

3. Process according to claim 2, **characterised in that** the separating-out of the portions of yarn takes place by counting them.

4. Process according to claim 2, **characterised in that** the separating-out of the portions of yarn takes place by cutting-out.

5. Process according to claim 1, **characterised in that** certain foreign substances are correlated with certain classes.

6. Process according to claim 1, **characterised in that** the yarn (8) is optically scanned by incident light.

7. Device for performing the process according to claim 1, having a sensor (1) for scanning the surface of the yarn (8), an arrangement for outputting an electrical signal (10) and an evaluating unit (2) in which a number of threshold values, in each case, for the length of a portion of yarn are stored and in which a comparison between a value of the electrical signal and all the threshold values takes place continuously, **characterised by** a sensor (1) which operates optically and in which a signal is obtained through the reflection of light on the yarn, and by threshold values for lengths and diameters which constitute a measure of the reflection on the yarn, the evaluating unit being constructed and disposed for determining types of foreign substances in the yarn, starting from their classification.

8. Device according to claim 7, **characterised in that** the sensor is disposed directly at a spinning point.

9. Device according to claim 7, **characterised in that** the sensor is disposed directly at a spooling point.

## Revendications

1. Procédé pour surveiller la qualité de fils, où un signal dérivé d'un fil est classé dans un champ de classification, **caractérisé en ce qu'**un signal de réflexion (10) dérivé du fil (8) est classé dans le champ de classification (16) et **en ce que**, en partant de cette classification, des sortes de matières étrangères sont identifiées dans le fil.

2. Procédé selon la revendication 1, **caractérisé en ce que** pour des valeurs du signal électrique, un champ de classification (16) est prédéfini qui présente respectivement plusieurs valeurs seuil (25 à 33 et 20 à 25) pour l'amplitude et la longueur/durée d'un segment du signal électrique, **en ce que** le signal est comparé avec toutes les valeurs seuil, **en ce que** des classes (34) sont formées par des combinaisons de quatre valeurs seuil, **en ce que** poux chaque segment de fil, l'appartenance à une classe est déterminée et **en ce que**, en partant de l'appartenance aux classes, des segments de fil de la même classe sont éliminés.

3. Procédé selon la revendication 2, **caractérisé en ce que** l'élimination des segments de fil a lieu par leur comptage.

4. Procédé selon la revendication 2, **caractérisé en ce que** l'élimination des segments de fil a lieu par découpe.

5. Procédé selon la revendication 1, **caractérisé en ce que** des classes déterminées sont associées à des matières étrangères déterminées.

6. Procédé selon la revendication 1, **caractérisé en ce que** le fil (8) est exploré d'une manière optique par une lumière incidente.

7. Dispositif pour la mise en oeuvre du procédé selon la revendication 1, avec un capteur (1) pour explorer la surface du fil (8), une installation pour émettre un signal électrique (10) et une unité d'évaluation (2) dans laquelle sont stockées respectivement plusieurs valeurs seuil pour la longueur d'un segment de fil et dans laquelle a lieu continuellement une comparaison entre une valeur du signal électrique et toutes les valeurs seuil, **caractérisé par** un capteur (1) fonctionnant d'une manière optique, par lequel un signal est obtenu par une réflexion de la lumière au fil, par des valeurs seuil pour des longueurs et diamètres qui constituent une mesure pour la réflexion au fil, où l'unité d'évaluation est réalisée et disposée pour déterminer des sortes de matières étrangères dans le fil en partant de leur classification.

8. Dispositif selon la revendication 7, **caractérisé en ce que** le capteur est disposé directement à un emplacement de filage.

9. Dispositif selon la revendication 7, **caractérisé en ce que** le capteur est disposé directement à un emplacement de bobinage.
